# EUROPEAN PATENT APPLICATION

(11) **EP 4 671 765 A1**
(43) Date of publication of application: **31.12.2025**
(21) Application number: 23958532.6
(22) Date of filing: 14.11.2023
(51) Int. Cl.: G01N 33/68

(54) **METHOD, SYSTEM, COMPOSITION AND KIT FOR DIAGNOSIS AND DIFFERENTIAL DIAGNOSIS OF ALZHEIMER'S DISEASE BASED ON HUMAN BRAIN HIPPOCAMPUS SPATIAL TRANSCRIPTOMICS**

(71) Applicant: Zhejiang University, Hangzhou, Zhejiang 310009 (CN); The First Affiliated Hospital, Zhejiang University School of Medicine, Hangzhou, Zhejiang 310009 (CN)
(72) Inventor: ZHANG, Jing, Hangzhou, Zhejiang 310009 (CN); WANG, Pan, Hangzhou, Zhejiang 310009 (CN); GUO, Zhen, Hangzhou, Zhejiang 310009 (CN); XU, Zhi, Hangzhou, Zhejiang 310009 (CN); HAN, Lei, Hangzhou, Zhejiang 310009 (CN); PAN, Danhua, Hangzhou, Zhejiang 310009 (CN)
(74) Representative: Bayramoglu et al.
(86) International application number: PCT/CN2023/131661
(87) International publication number: WO 2025/102250

(57) **Abstract**

The present disclosure discloses a method, system, composition, and kit for diagnosis and differential diagnosis of Alzheimer's disease (AD) based on spatial transcriptomics of a human hippocampus. In the present disclosure, the rapid and efficient early diagnosis and differential diagnosis of AD-associated cognitive impairment is achieved based on one or more of cholecystokinin (CCK), neurogranin (NRGN), and peripheral myelin protein 2 (PMP2) that are carried by plasma extracellular vesicles (EVs). The present disclosure enables the high-sensitivity and high-throughput detection of nervous system-derived EVs in peripheral blood, and demonstrates advantages such as high efficiency and low cost. The present disclosure provides a new technical means and method for the clinical application and large-scale screening related to the accurate diagnosis of AD-associated cognitive impairment.

## Description

### TECHNICAL FIELD

The present disclosure belongs to the technical field of accurate diagnosis of Alzheimer's disease (AD), and relates to a method, system, composition, and kit for diagnosis and differential diagnosis of AD based on spatial transcriptomics of a human hippocampus. In particular, the present disclosure relates to acquisition of a method, system, composition, and kit for diagnosis and differential diagnosis of AD based on data of research and development on spatial transcriptomics/single-cell sequencing of a human hippocampus and screening of the data in combination with an exosome detection technique.

### BACKGROUND TECHNOLOGY

The technology for early diagnosis of AD remains immature, and there is a limited variety of products related to the early diagnosis of AD. Because there is currently a lack of specific and sensitive early diagnosis approaches and criteria for AD in clinical practice, AD patients are often diagnosed based on clinical symptoms and imaging indicators. However, when AD is diagnosed in this way, AD patients have already experienced substantial irreversible neuronal damage, and AD has progressed to the middle to late stages, missing the optimal window for intervention. Moreover, there is currently a lack of rapid and efficient diagnostic methods to distinguish between AD and non-AD cognitive impairment. The pathogenesis of AD has not been clarified in the current research. As a result, there are clinically no effective drugs for the etiological treatment of AD, and only the symptomatic treatment to alleviate the symptoms of AD patients is available.

In clinical practice, imaging techniques hold a specified value for the diagnosis of AD. However, imaging examinations are extremely costly, and only large general hospitals are equipped with the relevant instruments for imaging examinations. Moreover, an imaging examination process is complex and time-consuming, and requires highly-experienced radiologists for diagnosis. Consequently, the imaging examinations pose a significant economic burden to both patients and society. Compared to the imaging examinations involving high costs and complex procedures, the detection based on AD biomarkers offers advantages such as low cost and low time consumption. Cerebrospinal fluid (CSF)-related biomarkers have been found to possess a specified clinical value for assisting in the diagnosis of AD. However, the collection of CSF requires high-level professional skills from medical staff, and patients often demonstrate low acceptance for the invasive and risky lumbar puncture. Thus, the collection of CSF is extremely challenging in clinical diagnosis, particularly in clinical screening efforts.

As previously mentioned, the early diagnosis and differential diagnosis of AD-associated cognitive impairment have long been challenges and research bottlenecks in both clinical practice and scientific research. Diagnostic biomarkers for AD developed based on peripheral blood have huge advantages including minimal invasiveness, high patient acceptance, simple operations, easy standardization, low cost, and easy popularization, and are of great significance for clinical practice and public health.

### CONTENT OF THE INVENTION

In order to overcome the shortcomings in the prior art, the present disclosure provides a method, system, composition, and kit for diagnosis and differential diagnosis of AD based on data of research and development on spatial transcriptomics/single-cell sequencing of a human hippocampus and screening of the data.

In the present application, primarily based on data of spatial transcriptomics/single-cell sequencing of a human brain in combination with an exosome database, candidate targets are screened, and nervous system-derived, brain region-specific extracellular vesicles (EVs) in peripheral blood are detected, so as to achieve the early diagnosis and differential diagnosis of AD. Due to advantages such as minimal invasiveness, high patient acceptance, low cost, and easy popularization, the present disclosure is expected to be used in clinical practice and large-scale screening. The key biomarkers in the present application are the differential markers discovered based on the spatial transcriptomics of a human brain. Due to disease specificity and brain region specificity, these key biomarkers can directly reflect the central nervous system-specific pathological changes related to AD, thereby achieving the diagnosis and differential diagnosis of AD. In the present application, the screening is conducted with reference to all differentially-expressed genes (A2M, ABCA2, ABI1, AC009879.3, AC091167.2, AC092143.1, AC093330.1, APOE, AC093512.2, ACADVL, ACAT2, ACTB, ACTG1, ACTR1A, ADD3, ADGRB3, ADGRG1, ADIRF, AEBP1, AHNAK, C9orf16, AHSA1, AL049839.2, AL121594.1, AL365205.1, ANGPTL4, ANK2, AQP1, AP2M1, C3, AP2S1, APLNR, APOC1, ARL1, ARL6IP1, ARPC2, ARPC5L, CALM1, ATF4, CABP7, CADM2, ATP1A1, ATP1B1, BHLHE22, ATP2A2, ATP5F1A, ATP5F1B, CALM3, ATP5F1D, ATP5F1E, CADM3, ATP5MC1, ATP5MC2, ATP5MC3, ATP5MF, CACYBP, ATP5MG, ATP5MPL, CALM2, ATP5PB, ATP6AP2, ATP6V0E2, ATP6V1D, ATP6V1E1, ATP6V1F, ATP6V1H, BEX4, ATP8A1, AUTS2, AUXG01000058.1, B2M, B4GAT1, BAIAP3, BCYRN1, BEX1, C1orf216, BEX2, BEX3, BRK1, C1QB, BSCL2, BTBD3, BTF3, CALY, CAMK1, CAMK2B, CAMK2N1, CAPS, CBR1, CCT5, CCT8, CD14, CD151, CD44, CD59, CD63, CD74, CEBPD, CELF4, CELSR2, CHGB, CHI3L1, CFL1, CHCHD2, CELF2, CD81, CD9, CDK2AP1, CDKN1A, CIRBP, CLSTN1, CLSTN2, CNN3, CKB, COMT, CLDN5, CLU, CNBP, CLASP2, CLDN11, CNDP2, COPA, DLG2, COX4I1, COX6C, COX5A, COX5B, COX6A1, COX6B1, COX7A2, COX7C, CRYAB, CSMD1, CSMD3, CYCS, DBI, CSRP1, CST3, CTSB, CTSD, CTXND1, CX3CL1, DGKG, DHCR24, DHCR7, DKK3, DCLK1, DDIT4, DEGS1, DNAJA1, DNAJA4, DNPH1, DSE, DTD1, DUSP1, DYNLL1, EEF1A1, EEF2, EEF1G, EFHD1, EID1, EIF4A1, EIF4A2, EIF5, ELOB, ENC1, ENO1, ENO2, FBXO2, EPDR1, EPHA4, EPHA5, ERBIN, ERC2, ERH, FABP3, FABP5, FAM107A, FAM162A, FAM3C, FAU, FBXW5, FEZ1, FHL1, FIS1, FKBP1B, FKBP2, FKBP5, FOS, FSCN1, FTH1, FTL, FTX, GALNT15, FXYD6, GABARAP, GABARAPL1, GABARAPL2, GADD45B, GAPDH, GDF1, GDI1, GLS, GLUL, GNAI2, GNAO1, GNAS, GNB2, GOT1, GPM6B, GPRC5B, GRB2, GRIA1, GSN, GSTP1, GUK1, H2AFZ, H3F3B, HACD3, HINT1, HLA-B, HLA-DRB1, HMGCR, HMGCS1, HNRNPC, HNRNPDL, HNRNPK, HOPX, HS6ST3, HSP90AA1, HSP90AB1, HSPA4, HSPA8, HSPB1, HSPB8, HSPE1, HSPH1, HTRA1, ICAM5, ID4, IDI1, ISCU, ISG15, ITGAV, IDS, ITGB4, ITM2B, ITM2C, JUN, ITPKB, IGFBP5, IGFBP7, IRS2, JUNB, JUND, KCNIP4, KCNMB4, KIAA0408, KIF1A, KIF1B, KLF9, KLHL2, LARP6, LDHA, LDHB, LGALS1, MAPK8IP3, MIR7-3HG, MICAL2, MGST3, MAP4K4, LINC00844, MARCKSL1, MFSD6, MFSD4A, LMO4, LRP1B, LSAMP, MAL, MALAT1, MAOB, MGST1, MAP1LC3A, MDH2, MAP1LC3B, MAP2K1, MDH1, MEG3, MLLT11, MMD, MRFAP1, MRPL41, MRPL51, MRPS21, MSMO1, MT1E, MT2A, MTATP6P1, MTCH1, MT-CO2, MT-CO3, MT-CYB, MTLN, MT-ND1, MT-ND2, MT-ND4, MT-ND6, MTPN, MT-RNR1, MT-RNR2, MYL12B, NDUFA11, MYL6, NDUFA13, NAA60, NDUFA4, NACA, NAP1L5, NAPB, NCAM2, NCDN, NDRG1, NDRG3, NDRG4, NDUFA1, NDUFA8, NDUFB8, NDUFAB1, NDUFS6, NDUFAF8, NDUFB4, NDUFV1, NEAT1, NECAB1, NENF, NLRP1, NME1, NME1-NME2, NOP56, NORAD, NPC2, NPM1, NPTN, NPTXR, NRIP3, NRN1, NRXN1, NRXN3, NTRK2, NUCKS1, NUPR1, OAZ1, OLFM1, OXCT1, PREPL, PAK3, PARK7, PCDH8, PCDH9, PCP4, PDHA1, PEBP1, PEX5L, PFKP, PGAM1, PGK1, PGRMC1, PHC2, PHPT1, PI4KA, PIP4K2A, PITHD1, PKM, PLCB1, PLEC, PLEKHA2, PLLP, PRDX5, POLR2I, PPIA, PRDX1, PPP2CA, PPP2R2B, PPP3CA, PPP3R1, PPT1, PREX1, PSMA7, PRKAR1B, PRKCA, PRXL2A, PSMA4, PSAP, PSMB4, PSMB5, PSMB7, PSMC3, PSMD1, PSMD8, PTMA, PTMS, PTP4A2, PTPRD, PTTG1IP, QDPR, QKI, RAB31, RABAC1, RACK1, RPL18A, RAMP1, RAN, RANGAP1, RASD1, RASGRP1, RBM3, RBX1, RFK, RGMA, RGS14, RPL18, RHBDD2, RNASE1, RNASET2, RPL10, RPL17-C18orf32, RPL10A, RPL11, RPL13, RPL13A, RPL15, RPL17-C18orf32, RPL19, RPL23, RPL21, RPL27A, RPL27, RPL28, RPL24, RPL26, RPL3, RPL30, RPL31, RPL34, RPL35, RPL35A, RPL36, RPL37A, RPL38, RPL4, RPL41, RPL5, RPL8, RPL9, RPLP0, RPLP1, RPS10, RPS11, RPS12, RPS13, RPS14, RPS15, RPS15A, RPS16, RPS19, RPS2, RPS20, RPS21, RPS23, RPS27, RPS27A, RPS29, RPS3, RPS3A, RPS4X, RPS6, RPS7, RPS8, RPSA, RTN3, RTN4, RYR2, S100A10, S100A6, SAP18, SAT1, SCAMP5, SCD, SCG3, SCGN, SCOC, SCRG1, SDHA, SEC61B, SELENOP, SELENOW, SEM1, SEMA3B, SEMA5A, SERF2, SERINC1, SHTN1, SIK3, SKP1, SLC22A17, SLC24A3, SLC25A3, SLC25A4, SLC2A3, SLC38A2, SLC44A1, SLC7A11, SLIT1, SNRPN, SOD1, SOD2, SORBS1, SPARC, SPARCL1, SPOCK1, SPP1, STXBP1, SUB1, SUMO2, SUN2, SUSD4, SYNE1, SYNM, SYS1-DBNDD2, SYT11, SYT13, TAGLN3, TALDO1, TBCA, TCEAL2, TCEAL3, TCEAL4, TCEAL5, TCF4, TCP1, TENM2, TF, THY1, TIMP2, TIMP3, TKT, TM2D3, TM9SF2, TMA7, TMBIM1, TMEM130, TMEM35A, TMSB10, TMSB4X, TMTC1, TNRC6C, TOLLIP, TOMM34, TOMM7, TPI1, TPPP, TPT1, TRIM2, TRMT112, TSC22D1, TSPAN7, TSPYL4, TUBA1A, TUBA4A, TUBB2A, TXN, TXNIP, TXNL1, UBB, UBC, UBE2N, UBL5, UBQLN1, UCHL1, UQCR10, UQCR11, UQCRB, UQCRH, UQCRQ, USP11, USP22, VAMP2, VAPA, VDAC2, VIM, VSTM2L, WBP2, XRCC6, YJEFN3, YWHAB, YWHAG, YWHAH, YWHAQ, YWHAZ, ZCCHC12, ZCCHC24, ZFAND5, ZFP36, ZFP36L1, ZFP36L2, and ZMAT2) from data of spatial transcriptomics/single-cell sequencing of a human hippocampus and an exosome database to identify genes (AK5, ALDOC, AMER, APC2, APOD, ATP1A3, BAALC, BCAN, C1orf61, CABP1, CACNG3, CACNG8, CAMKV, CCK, CHN1, CNKSR2, CHN1, CNDP1, CNTNAP4, CREG2, CTXN1, DLGAP1, DNAJC6, ENHO, GABRA5, GAD1, GAP43, GFAP, GNG3, GPM6A, GRIK2, GRIN1, GRIN2A, GRIN2B, HEPACAM, HPCA, KCNJ10, KCNQ3, KCTD16, KIF5C, LAMP5, MAP2, MBP, MOBP, MTURN, MLC1, MT3, MTURN, NEFL, NEFM, NEUROD2, NKX6-2, OMG, NPTX1, NRGN, OLIG1, OPALIN, PAQR6, PDYN, PHYHIP, PIANP, PLANP, PLEKHB1, PLP1, PMP2, PMP22, PNMA2, POLR2F, PPKCG, PTPRZ1, RAB3A, RGS4, RTN1, S100B, SCN2B, SEZ6L, SLC1A2, SLC17A7, SLC1A3, SLC24A2, SLC2A1, SLC4A10, SLC6A7, SNAP25, SNCB, STMN4, STMN2, SULT4A1, SYN2, SYNPR, SYT1, TTC9B, TUBB2B, and TUBB4A) that change significantly during the occurrence and development of AD. The resulting data is reliable and can further enhance the reliability of diagnosis and differential diagnosis.

To achieve the above objective, the present disclosure provides a composition for diagnosis and differential diagnosis of AD based on research and development of spatial transcriptomics/single-cell sequencing of a human hippocampus, including one or more of cholecystokinin (CCK), neurogranin (NRGN), and peripheral myelin protein 2 (PMP2) that are carried by plasma EVs.

Preferably, the CCK, the NRGN, and the PMP2 that are carried by the plasma EVs are enriched through PEG8000 precipitation.

The present disclosure provides a test kit, including a reagent Zenon^{™} Alexa Fluor^{™} 647 Rabbit IgG Labeling kit for labeling an antibody, a lipid probe, and the antibody,
where a sequence of the lipid probe is shown in SEQ ID NO: 1: TTTTTTTTTTTTTTTTTTTTTTTTTTTTTT, with a 5'-terminus modification of 5' 6-CY3 and a 3'-terminus modification of 3'Cholesteryl; and the antibody includes one or more of anti-CCK antibody, anti-NRGN antibody, and anti-PMP2 antibody.

The present disclosure provides a use of a CCK protein in differential diagnosis of AD and a non-AD dementia.

The present disclosure provides a method for detecting positive proteins CCK, NRGN, and PMP2, including the following steps:
labeling anti-CCK antibody, anti-NRGN antibody, and anti-PMP2 antibody with an Alexa Fluro fluorescent labeling kit; thoroughly mixing the labeled antibodies with completely-blocked exosomes, and incubating overnight at 4°C in the dark; adding the lipid probe, and incubating at 4°C in the dark;
adding PFA to a labeled sample, thoroughly mixing, and incubating at room temperature in the dark;
diluting with phosphate buffered saline (PBS) to an appropriate concentration, loading on CytoFLEX or a similar nano-flow cytometry instrument, and analyzing at an event rate of less than 10,000 events per second; and
in a VSSC mode of the CytoFLEX or the similar nano-flow cytometry instrument, determining a positive rate of a protein maker under a lipid probe-positive condition.

Preferably, the method is used to achieve identification for a non-disease diagnosis and treatment purpose.

The present disclosure has the following beneficial effects:
The core detection technology adopted in the present application is nano-flow cytometry. The nano-flow cytometry can achieve the high-sensitivity and high-throughput detection of central nervous system-derived EVs in peripheral blood, and demonstrates advantages such as high efficiency and low cost, which provides a technical support for clinical application and large-scale screening. The present application is intended to address the clinical and scientific research challenges of early diagnosis and differential diagnosis of AD-associated cognitive impairment. Through the world-leading spatial transcriptomics and single-cell sequencing technologies and the fire-new nano-flow cytometry technology, the present application innovatively identifies central nervous system-derived, brain region-specific EVs markers from Chinese human brain resources. The present application enables the rapid and efficient early diagnosis and differential diagnosis of AD-associated cognitive impairment, and provides a novel technical means and method for clinical diagnosis of AD-associated cognitive impairment.

### DESCRIPTION OF THE DRAWINGS

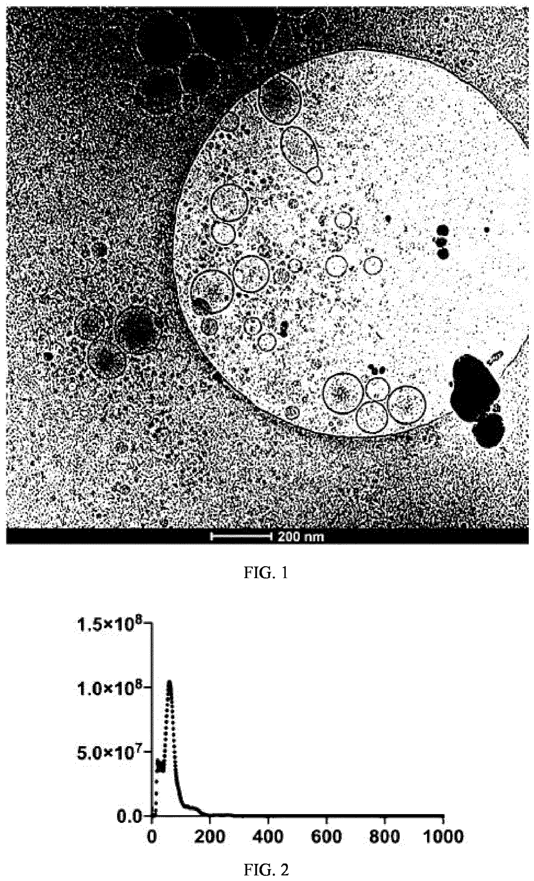

FIG. 1 is a transmission electron microscopy image for the identification of plasma EVs extracted in Example 1 of the present disclosure, where the transmission electron microscopy image indicates that the plasma EVs have a bilayer membrane structure and possess a particle size in a typical particle size range for EVs;
FIG. 2 shows particle size distribution and concentration analysis results of EVs enriched through PEG8000 precipitation in Example 1 of the present disclosure, where an x-coordinate represents a particle size distribution and a y-coordinate represents a concentration of EVs;

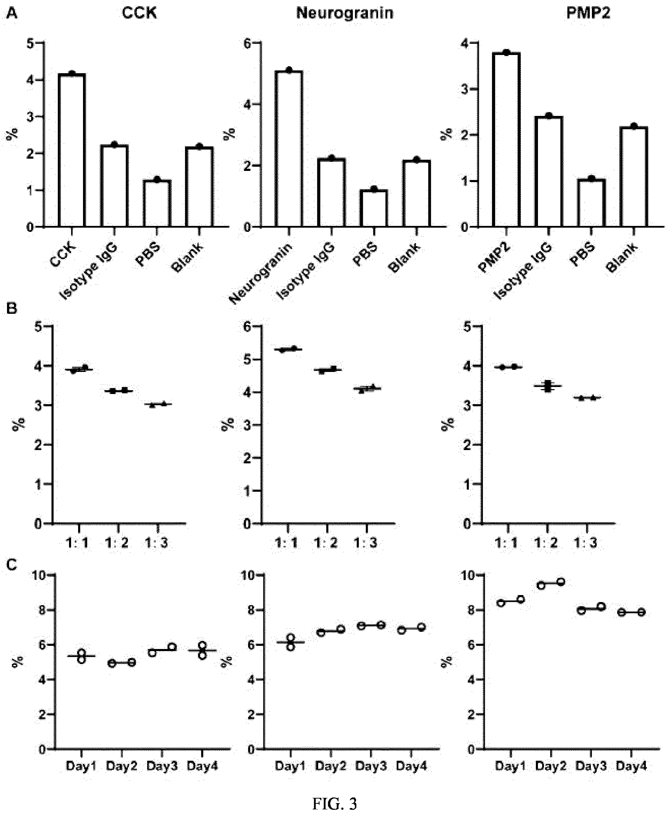

FIG. 3 shows nano-flow cytometry test results of specificity and stability of the plasma EVs extracted in Example 1 of the present disclosure, where A shows that percentages of EVs carrying CCK, NRGN, and PMP2 in peripheral plasma are significantly higher than an isotype IgG in a control group; and B and C show that labeling percentages of anti-CCK antibody, anti-NRGN antibody, and anti-PMP2 antibody for EVs remain stable under varying dilution factors (B) and varying incubation times (C);

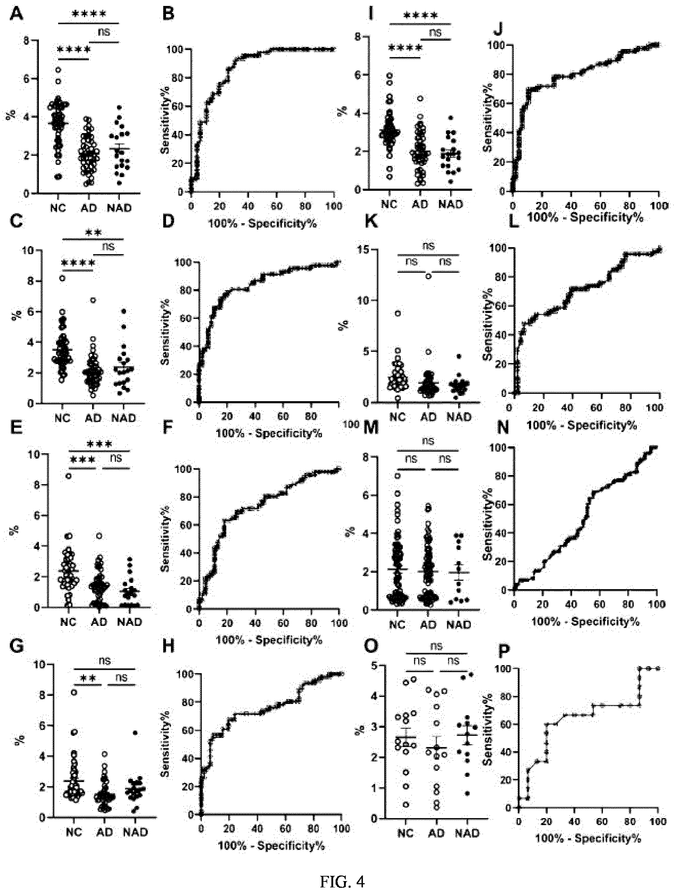

FIG. 4 shows screening of differential diagnosis abilities of some potential biomarkers in the present disclosure, where A and B show statistical analysis and receiver operating characteristic (ROC) results for CCK protein detection; C and D show statistical analysis and ROC results for NRGN protein detection; E and F show statistical analysis and ROC results for PMP2 protein detection; G and H show statistical analysis and ROC results for CREG2 protein detection; I and J show statistical analysis and ROC results for CPLX2 protein detection; K and L show statistical analysis and ROC results for NEFM protein detection; M and N show statistical analysis and ROC results for STMN4 protein detection; and O and P show statistical analysis and ROC results for STMN protein detection; and

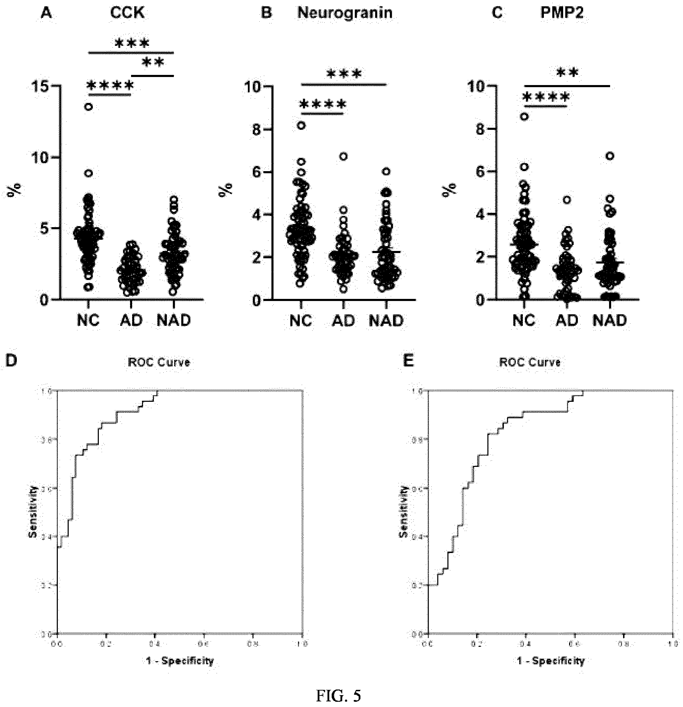

FIG. 5 shows analysis charts of verification queue results in Example 3 of the present disclosure, where as shown in FIG. 5A, a proportion of CCK-positive EVs in total EVs in plasma of an AD group is significantly reduced compared to a normal control (NC) group (****, p < 0.000), a proportion of CCK-positive EVs in total EVs in plasma of the AD group is also significantly reduced compared to an NAD group (***, p < 0.001), and a proportion of CCK-positive EVs in total EVs in plasma of the NAD group is also significantly reduced compared to the NC group (**, p < 0.01); as shown in FIG. 5B, a proportion of NRGN-positive EVs in total EVs in plasma of the AD group is significantly reduced compared to the NC group (****, p < 0.0001), and a proportion of NRGN-positive EVs in total EVs in plasma of the NAD group is also significantly reduced compared to the NC group (***, p < 0.001); as shown in FIG. 5C, a proportion of PMP2-positive EVs in total EVs in plasma of the AD group is significantly reduced compared to the NC group (****, p < 0.0001), and a proportion of PMP2-positive EVs in total EVs in plasma of the NAD group is also significantly reduced compared to the NC group (**, p < 0.01); as shown in FIG. 5D, CCK, NRGN, and PMP2 data is incorporated by the Enter method of logistic regression analysis, with AUC = 0.92 (NC VS AD); and as shown in FIG. 5E, CCK, NRGN, and PMP2 data is incorporated by the Enter method of logistic regression analysis, with AUC = 0.83 (AD VS NAD).

### SPECIFIC IMPLEMENTATIONS

To well illustrate the objective, technical solutions, and advantages of the present disclosure, the present application will be further explained below in conjunction with specific examples.

### Example 1

Enrichment of EVs through PEG8000 precipitation
1. Plasma from a subject was rapidly thawed at 37°C (within 2 min) and vortexed for thorough mixing.
2. A resulting plasma sample (larger than 300 µL) was centrifuged at 4°C and 2,000 × g for 15 min, and a resulting supernatant was collected.
3. The supernatant was further centrifuged at 4°C and 12,000 × g for 30 min, and a resulting plasma supernatant was collected.
4. 10 µL of the plasma supernatant was pipetted and transferred to a 1.5 mL centrifuge tube, and then 70 µL of PBS and 20 µL of 40% PEG8000 were added. A resulting mixture was fully pipetted up and down for thorough mixing, and allowed to stand at room temperature for 30 min.
5. Centrifugation was conducted at 12,000 g and 4°C for 20 min.
6. A resulting supernatant was discarded, and 100 µL of PBS was added for resuspending. A resulting system was pipetted up and down for thorough mixing, dispensed at 10 µL/tube, and stored at -80°C for later use.

### Example 2

1. Sample blocking
   (1) 10 µL of EVs enriched through PEG8000 precipitation according to the method above (Example 1) was taken and added to a 0.6 mL centrifuge tube.
   (2) 10 µL of a 2% BSA solution (filtered through a 0.22 µm filter membrane) was added, and thorough mixing was conducted.
   (3) Blocking was conducted at room temperature (25°C to 26°C) for 1 h to eliminate nonspecific binding.
   (4) 10 µL of PBS (filtered through a 0.22 µm filter membrane) was added for dilution to terminate the blocking.
2. Sample labeling
   Antibodies for detecting biomarkers were labeled by a labeling method. Anti-CCK antibody, anti-NRGN antibody, and anti-PMP2 antibody were labeled with an Alexa Fluro fluorescent labeling kit. The reagent for labeling the antibodies was a Zenon^{™} Alexa Fluor^{™} 647 Rabbit IgG Labeling Kit purchased from Thermo Fisher Scientific. A probe for labeling a lipid had a sequence shown in SEQ ID NO: 1: TTTTTTTTTTTTTTTTTTTTTTTTTTTTTT, with a 5'-terminus modification of 5'6-CY3 and a 3'-terminus modification of 3'Cholesteryl.
   (1) 1 µg of an antibody for a corresponding marker was taken and diluted with PBS (filtered through a 0.22 µm filter membrane) to 5 µL (0.2 µg/µL).
   (2) 5 µL of a Zenon^{™} AlexaFluor^{™} 647 Rabbit IgG Labeling Kit solution A was added, thorough mixing was conducted, and incubation was conducted at room temperature (25°C to 26°C) in the dark for 20 min.
   (3) 3 µL (3 µg) of a Zenon^{™} Alexa Fluor^{™} 647 Rabbit IgG Labeling Kit solution B (the solution B was diluted to 1 µg/µL) was added to a solution produced in the step (2), thorough mixing was conducted, and incubation was conducted at room temperature (25°C) in the dark for 10 min to quench the unbound free fluorescein.
   (4) Dilution was conducted with PBS (filtered through a 0.22 µm filter membrane) to a total volume of 50 µL.
   (5) 3 µL of a Zenon^{™} Alexa Fluor^{™} 647 Rabbit IgG Labeling Kit-labeled antibody was added to a sample blocked in the step 1, thorough mixing was conducted, and incubation was conducted overnight at 4°C in the dark.
3. Reagent for probe incubation: A DNA anchor was synthesized. A sequence of the DNA anchor was TTTTTTTTTTTTTTTTTTTTTTTTTTTTTT (SEQ ID NO: 1), with a 5'-terminus modification of 5'6-CY3 and a 3'-terminus modification of 3'Cholesteryl. This lipid probe could bind to the membrane of EVs. Probe-labeled positive particles were considered as actual EVs. This method further enhanced the scientificity and accuracy of EVs labeling.
   (1) The next day, 62 µL of PBS and 5 µL of the lipid probe (a concentration of a working solution of the lipid probe was 10 µM) were added (a reaction volume was 100 µL, and a final probe concentration was 500 nM), and incubation was conducted at 4°C in the dark for 1 h.
   (2) 100 µL of 4% PFA (filtered through a 0.22 µm filter membrane) was added to a labeled sample, thorough mixing was conducted, and incubation was conducted at room temperature (25°C to 26°C) in the dark for 20 min.
   (3) A resulting test sample was diluted with PBS to an appropriate concentration, loaded on CytoFLEX, and analyzed at an event rate of less than 10,000 events per second. 50,000 particles were collected within a lipid probe gate (in this case, there were approximately 100,000 particles in total, and in other words, when particles obtained in this example were screened with the lipid probe, approximately 50% were identified as actual EVs).
4. Detection

In a VSSC mode of CytoFLEX, a positive rate of a related protein maker was determined under a lipid probe-positive condition.

### Example 3

The differential diagnosis performance for screening of the above biomarkers was assessed in a small cohort.

Plasma samples were collected from 15 age- and gender-matched healthy controls (NC group), 15 subjects with AD (AD group), and 15 subjects with a non-AD dementia (NAD group). The experiment was conducted according to the methods in Examples 1 and 2.

Quantities of EVs labeled with fluorescent markers individually for CCK, NRGN, PMP2, CREG2, CPLX2, NEFM, STMN4, and STMN2 were determined in a VSSC mode of CytoFLEX, and its proportion among positive EVs labeled with the lipid probe was calculated. An ability of each biomarker to distinguish among NC, AD, and NAD was determined. Results were shown in FIG. 4.

A proportion of CCK-positive EVs in total EVs in plasma of the AD group was significantly reduced compared to the NC group (****, p < 0.0001), and a proportion of CCK-positive EVs in total EVs in plasma of the NAD group was significantly reduced compared to the NC group (****, p < 0.0001) (4A). ROC = 0.86 (4B).

A proportion of NRGN-positive EVs in total EVs in plasma of the AD group was significantly reduced compared to the NC group (****, p < 0.0001), and a proportion of NRGN-positive EVs in total EVs in plasma of the NAD group was significantly reduced compared to the NC group (**, p < 0.01) (4C). ROC = 0.84 (4D).

A proportion of PMP2-positive EVs in total EVs in plasma of the AD group was significantly reduced compared to the NC group (***, p < 0.001), and a proportion of PMP2-positive EVs in total EVs in plasma of the NAD group was significantly reduced compared to the NC group (**, p < 0.01) (4E). ROC = 0.80 (4F).

A proportion of CREG-2-positive EVs in total EVs in plasma of the AD group was significantly reduced compared to the NC group (**, p < 0.01) (4G). ROC = 0.76 (4H).

A proportion of CPLX2-positive EVs in total EVs in plasma of the AD group was significantly reduced compared to the NC group (****, p < 0.0001), and a proportion of CPLX2-positive EVs in total EVs in plasma of the NAD group was significantly reduced compared to the NC group (****, p < 0.0001) (4I). ROC = 0.79 (4J).

The AD group and the NAD group were not significantly different from the NC group in terms of a proportion of NEFM-positive EVs in total EVs in plasma (4K). ROC = 0.79 (4L).

The AD group and the NAD group were not significantly different from the NC group in terms of a proportion of STMN4-positive EVs in total EVs in plasma (4M). ROC = 0.69 (4N).

The AD group and the NAD group were not significantly different from the NC group in terms of a proportion of STMN2-positive EVs in total EVs in plasma (4O). ROC = 0.64 (4P).

### Example 4

Differential diagnosis abilities of the above biomarkers were evaluated in a clinical standard queue.

Plasma samples were collected from 66 age- and gender-matched healthy controls (NC group), 45 subjects with AD (AD group), and 45 subjects with a non-AD dementia (NAD group). The experiment was conducted according to the methods in Examples 1 and 2.

Quantities of EVs labeled with fluorescent markers individually for CCK, NRGN, and PMP2 were determined in a VSSC mode of CytoFLEX, and its proportion among positive EVs labeled with the lipid probe was calculated. An ability of each biomarker to distinguish among NC, AD, and NAD was determined. Results were shown in FIG. 5.

A proportion of CCK-positive EVs in total EVs in plasma of the AD group was significantly reduced compared to the NC group (****, p < 0.0001), and a proportion of CCK-positive EVs in total EVs in plasma of the AD group was also significantly reduced compared to the NAD group (**, p < 0.01), with significant differences (FIG. 5A).

A proportion of NRGN-positive EVs in total EVs in plasma of the AD group was significantly reduced compared to the NC group (****, p < 0.0001) (FIG. 5B). A proportion of PMP2-positive EVs in total EVs in plasma of the AD group was significantly reduced compared to the NC group (****, p < 0.0001) (FIG. 5C).

CCK, NRGN, and PMP2 data was incorporated by the Enter method of logistic regression analysis, with AUC = 0.92 (NC VS AD) (FIG. 5D). AUC = 0.83 (AD VS NAD) (FIG. 5E). According to the analysis results, proportions of single-labeled positive EVs for CCK, NRGN, and PMP2 in total EVs can not only effectively distinguish between NC and AD patients, but also allow the effective differential diagnosis of AD and NAD.

The above are merely the preferred embodiments of the present application and are not intended to limit the present application in any other form. Those skilled in the art may make changes or modifications based on the technical content disclosed above and the targets disclosed above to produce equivalent embodiments. Any simple modifications and equivalent changes and variations made to the above embodiments according to the technical essence of the present application without departing from the content of the technical solutions of the present application should fall within the protection scope of the technical solutions of the present application.

Finally, it should be noted that the above embodiments are provided merely to describe the technical solutions of the present disclosure, rather than to limit the protection scope of the present disclosure. Although the present disclosure is described in detail with reference to the preferred embodiments, a person of ordinary skill in the art should understand that modifications or equivalent replacements may be made to the technical solutions of the present disclosure without departing from the spirit and scope of the technical solutions of the present disclosure.

## Claims

1. A composition for diagnosis and differential diagnosis of Alzheimer's disease (AD) based on spatial transcriptomics of a human hippocampus, comprising one or more of cholecystokinin (CCK), neurogranin (NRGN), and peripheral myelin protein 2 (PMP2) that are carried by plasma extracellular vesicles (EVs).

2. The composition for the diagnosis and the differential diagnosis of the AD based on research and development on the spatial transcriptomics/single-cell sequencing of the human hippocampus according to claim 1, wherein the CCK, the NRGN, and the PMP2 that are carried by the plasma EVs are enriched through PEG8000 precipitation.

3. A test kit based on the composition according to claim 1 or 2, comprising a reagent Zenon^{™} Alexa Fluor^{™} 647 Rabbit IgG Labeling kit for labeling an antibody, a lipid probe, and the antibody,
wherein the sequence of the lipid probe is shown in SEQ ID NO: 1: TTTTTTTTTTTTTTTTTTTTTTTTTTTTTT, with a 5'-terminus modification of 5'6-CY3 and a 3'-terminus modification of 3'Cholesteryl; and
the antibody comprises one or more of CCK, NRGN, and PMP2.

4. A use of a CCK protein in differential diagnosis of AD and a non-AD dementia.

5. A method for detecting a positive protein with the composition according to claim 1 or 2 or a test kit according to claim 3, comprising the following steps:
labeling anti-CCK antibody, anti-NRGN antibody, and anti-PMP2 antibody with a Alexa Fluro fluorescent labeling kit; thoroughly mixing the labeled antibodies with completely-blocked exosomes, and incubating overnight at 4°C in the dark; and adding a lipid probe, and incubating at 4°C in the dark;
adding PFA to the labeled sample, thoroughly mixing, and incubating at room temperature in the dark;
diluting with phosphate buffered saline (PBS) to an appropriate concentration, loading on CytoFLEX, and analyzing at an event rate of less than 10,000 events per second; and
in a VSSC mode of the CytoFLEX, determining a positive rate of a protein maker under a lipid probe-positive condition.

6. The method according to claim 4, wherein the method is used to achieve identification for a non-disease diagnosis and treatment purpose.
